# EUROPEAN PATENT APPLICATION

(11) **EP 3 673 850 A1**
(43) Date of publication of application: **01.07.2020**
(21) Application number: 19219609.5
(22) Date of filing: 24.12.2019
(51) Int. Cl.: A61B 18/14, A61B 18/00, A61B 18/12

(54) **BALLOON CATHETER WITH DISTAL END HAVING A RECESSED SHAPE**

(30) Priority: 28.12.2018 US 201816236226
(71) Applicant: BIOSENSE WEBSTER (ISRAEL) LTD., 2066717 Yokneam (IL)
(72) Inventor: GOVARI, Assaf, 2066717 Yokneam (IL); BEECKLER, Christopher Thomas, Irwindale, CA California 91706 (US)
(74) Representative: Small, Gary James

(57) **Abstract**

A characterization system includes an inflatable balloon for insertion into a patient organ and one or more electrodes disposed on the balloon. When the balloon is in an inflated position, a distal end of the balloon has a recessed shape that orients respective regions of the electrodes perpendicularly to a longitudinal axis of the balloon.

## Description

### FIELD OF THE INVENTION

The present invention relates generally to medical devices, and particularly to methods and systems for ablating tissue using a balloon catheter with a distal end having a recessed shape.

### BACKGROUND OF THE INVENTION

Focal catheters are used in various medical procedures, such as in cardiac ablation procedures.

For example, U.S. Patent Application Publication 2014/0114304 describes an ablation therapy system comprising an ablation catheter system for treating atrial fibrillation (AF). The ablation catheter system comprises a catheter body including a lumen for receiving a visualization catheter, an ablation element for ablating tissue in a patient's heart having abnormal electrical activity, a support assembly for supporting the ablation element, the support assembly being supported by the catheter assembly.

U.S. Patent Application Publication 2014/0114304 describes an ablation catheter for ablating tissue. The catheter comprising a telescopic tubular body comprising an external tubular body and an internal tubular body concentric with each other, and a rod-like guiding element at least partly housed in the internal tubular body with at least one free end protruding from the internal tubular body in correspondence with a distal end of the telescopic body. The catheter further comprises a positioning head and an ablation head in correspondence with the distal end of the telescopic body, the positioning head being situated in the proximity of the free end of the rod-like guide, and the ablation head in the proximity of the positioning head, in a remote position with respect to the free end.

### SUMMARY OF THE INVENTION

An embodiment of the present invention that is described herein provides a characterization system including an inflatable balloon for insertion into a patient organ, and one or more electrodes disposed on the balloon. When the balloon is in an inflated position, a distal end of the balloon has a recessed shape that orients respective regions of the electrodes perpendicularly to a longitudinal axis of the balloon.

In some embodiments, the characterization system includes a power generator, which is configured to supply electrical power to each of the one or more electrodes. In other embodiments, the electrodes include at least a first electrode and a second electrode, and the power generator is configured to apply a first power level to the first electrode and a second different power level to the second electrode. In yet other embodiments, the characterization system includes a processor, which is configured to control power levels that the power generator respectively supplies to the one or more electrodes.

In an embodiment, when the distal end of the balloon makes physical contact with the organ, the respective regions of the electrodes, which are perpendicular to the longitudinal axis of the balloon, are configured to apply frontal ablation to a wall of the patient organ. In another embodiment, in the inflated position, the electrodes have additional regions oriented not perpendicularly to the longitudinal axis, and the additional regions of the electrodes are configured to conduct an oblique ablation to tissue of the patient organ. In yet another embodiment, the patient organ includes a heart, and the oblique ablation includes ablation of an ostium of a pulmonary vein (PV) of the heart.

In some embodiments, the balloon includes an inner lumen, and the balloon is configured to move along a guidewire inserted through the inner lumen. In other embodiments, the balloon is configured to fold along the longitudinal axis, to a deflated position.

There is additionally provided, in accordance with an embodiment of the present invention, a method including, in a catheterization system that includes an inflatable balloon for insertion into a patient organ, and one or more electrodes disposed on the balloon, such that when the balloon is in an inflated position, a distal end of the balloon has a recessed shape that orients respective regions of the electrodes perpendicularly to a longitudinal axis of the balloon, inserting the inflatable balloon into the patient organ. The balloon is inflated to the inflated position for attaching the balloon to tissue of the patient organ. The tissue of the patient organ is ablated.

There is further provided, in accordance with an embodiment of the present invention, a method for producing a catheterization system, the method includes producing an inflatable balloon for insertion into a patient organ, when the balloon is in an inflated position, a distal end of the balloon has a recessed shape. One or more electrodes on the balloon are disposed, such that the recessed shape orients respective regions of the electrodes perpendicularly to a longitudinal axis of the balloon.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a catheterization system, in accordance with an embodiment of the present invention;
Fig. 2A is a schematic, side view of a distal-end assembly of a catheter, in accordance with an embodiment of the present invention;
Fig. 2B is a schematic, top view of a distal-end assembly of a catheter, in accordance with an embodiment of the present invention; and
Fig. 3 is a schematic, side view of contours of a distal-end assembly of a catheter in inflated and deflated positions, in accordance with embodiments of the present invention.

### DETAILED DESCRIPTION OF EMBODIMENTS

### OVERVIEW

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%. In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator whereas "distal" indicates a location further away to the operator or physician.

Embodiments of the present invention that are described hereinbelow provide improved methods and systems for efficient ablation of a large area of cardiac tissue using a focal catheter whose distal end has a recessed shape. In some embodiments, in preparation for ablation, a focal catheter comprising an inflatable balloon is inserted into a patient organ, e.g., patient heart, in a deflated position. The balloon is then inflated, and ablation is performed using one or more electrodes that are disposed on the surface of the balloon.

In some embodiments, the focal catheter is operable within a catheterization system that comprises a processor and a power generator that provides electrical power to the electrodes under the processor control. In some embodiments, the processor is configured to produce an ablation plan for a selected tissue of the heart, based on electro-potential (EP) signals received from the patient heart. In other embodiments, the ablation plan is produced by the processor using any suitable method, or provided to the processor.

In some embodiments, the power generator is electrically coupled to the electrodes disposed on the balloon, and the processor controls the amount of power that the power generator supplies to each electrode, or to a group of electrodes, based on the ablation plan. In some embodiments, the power generator is configured to supply the same amount of electrical power to all of the electrodes. In other embodiments, the power generator distributes electrical power among the electrodes non-uniformly, i.e., the power generator supplies to at least two of the electrodes different respective amounts of power.

In some embodiments, when the balloon is in the inflated position, the distal-end of the balloon has a recessed shape that orients respective regions of the electrodes perpendicularly and more particularly orthogonally to a longitudinal axis of the balloon.

During the ablation procedure, the distal-end of the balloon is placed in contact with the tissue at the target location, and the regions of electrodes that due to the recessed shape are perpendicular (when viewed on the side) and more particularly orthogonal to the longitudinal axis of the balloon make contact with the tissue. Under the processor control, the power generator powers the electrodes to apply frontal ablation to a wall of the target tissue based on the predefined ablation plan that specifies various ablation parameters, such as ablation time, target temperature, the required contact force to the ablated tissue, and ablation power.

In some embodiments, certain regions of the electrodes on the balloon surface can be used for ablating tissue oriented not perpendicularly to the longitudinal axis of the balloon. For example, the catheter is configured to ablate the ostium of a pulmonary vein (PV) so as to carry out a PV isolation procedure using regions of the electrodes that are oriented non-perpendicularly to the longitudinal axis of the balloon.

The disclosed techniques improve the ablation quality and reduce the cycle time of cardiac ablation procedures. The disclosed techniques improve the control of ablation parameters, such as contact force between the catheter and the ablated tissue, by having the longitudinal axis of the ablation catheter oriented perpendicularly (or more specifically, orthogonal) to the ablated tissue. The recessed shape of the distal end and the flexibility in supplying different power to different electrodes, enables to ablate a large tissue area concurrently with a required ablation pattern, which reduces the cycle time of the ablation procedure. Moreover, the disclosed techniques enable ablating various shapes of tissue in various locations of the patient's heart by using, for each shape, different regions of the electrodes disposed on the outer surface of the balloon.

### SYSTEM DESCRIPTION

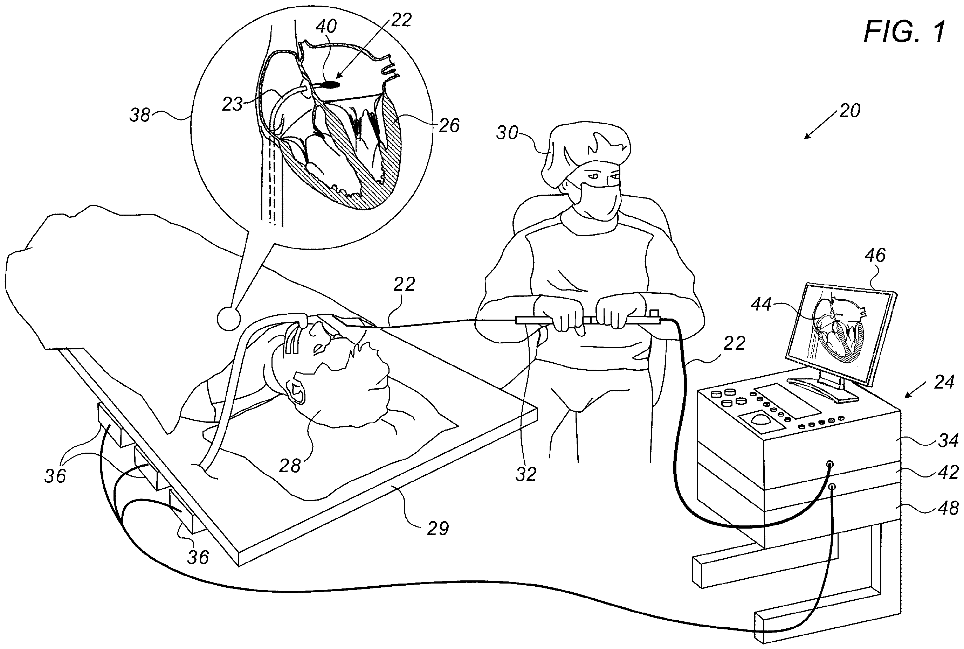

Fig. 1 is a schematic, pictorial illustration of a catheterization system 20, in accordance with an embodiment of the present invention. System 20 comprises a catheter, in the present example an inflatable balloon catheter 22 used in cardiac ablation procedures and/or in other minimally invasive medical applications.

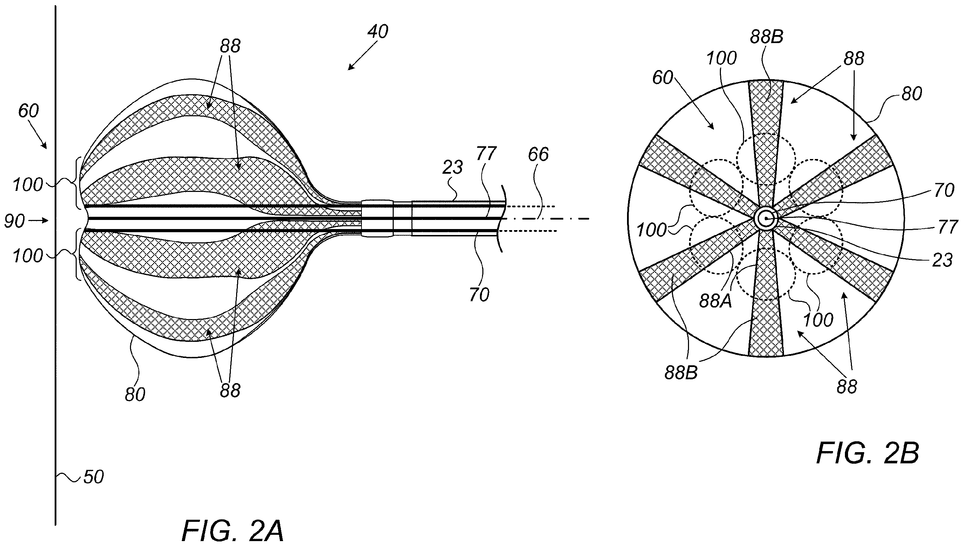

In the embodiment described herein, balloon catheter 22, also referred to herein as catheter 22 for brevity, may be used for any suitable therapeutic and/or diagnostic purposes, such as for sensing electro-potential signals or for ablating tissue of a heart 26 of a patient 28. The tissue ablation procedure, which is carried out using catheter 22, is described in more details in Figs. 2A and 2B below.

In some embodiments, system 20 comprises a control console 24, typically a general-purpose computer, with suitable front end and interface circuits for receiving signals from catheter 22. In some embodiments, control console 24 comprises a processor 34, configured to receive the signals from catheter 22 and to control other components of system 20 that are described herein.

In some embodiments, console 24 further comprises a display 46, which is configured to display data, such as an image 44 of at least part of heart 26 of patient 28. In some embodiments, image 44 may be acquired using any suitable anatomical imaging system, such as computerized tomography (CT), fluoroscopic imaging, or magnetic resonance imaging (MRI).

During the medical procedure, a physician 30, inserts catheter 22 through the vascular system of patient 28 lying on a table 29.

Reference is now made to an inset 38. In some embodiments, catheter 22 comprises a shaft 23 for navigating catheter 22 to a target location in heart 26. In some embodiments, shaft 23, or any other suitable component of catheter 22, is coupled to a distal-end assembly 40, depicted in detail in Figs. 2A and 2B below.

During the procedure, physician 30 moves distal-end assembly 40 in the vicinity of the target region in heart 26 by manipulating shaft 23 of catheter 22 using a manipulator 32 coupled near the proximal end of catheter 22. The proximal end of catheter 22 is connected to interface circuitry of processor 34.

In some embodiments, the position of distal-end assembly 40 in the heart cavity is typically measured using position sensing techniques. This method of position sensing is implemented, for example, in the CARTO™ system, produced by Biosense Webster Inc. (Irvine, Calif.) and is described in detail in U.S. Patents 5,391,199, 6,690,963, 6,484,118, 6,239,724, 6,618,612 and 6,332,089, in PCT Patent Publication WO 96/05768, and in U.S. Patent Application Publications 2002/0065455 A1, 2003/0120150 A1 and 2004/0068178 A1, whose disclosures are all incorporated herein by reference.

In some embodiments, console 24 further comprises a driver circuit 42, which drives magnetic field generators 36 placed at known positions external to patient 28, e.g., below the patient's torso.

In some embodiments, console 24 comprises a power generator 48, also referred to herein as a power supply unit (PSU), which is configured to supply power to various components of distal-end assembly 40. Processor 34 is configured to determine the amount of power supplied, by power generator 48, to each component of distal-end assembly 40, as will be depicted in Figs. 2A and 2B below.

In some embodiments, processor 34 is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

### TISSUE ABLATION USING A DISTAL-END HAVING RECESSED SHAPE

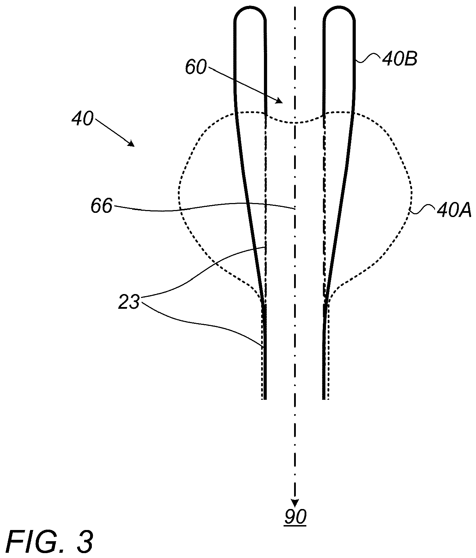

Fig. 2A is a schematic, side view of distal-end assembly 40, in accordance with an embodiment of the present invention. In some embodiments, distal-end assembly 40 comprises an inflatable balloon 80, which is configured to expand to an inflated position, as shown in Fig. 2A, and to collapse to a deflated position, as will be described in detail in Fig. 3 below.

In some embodiments, when balloon 80 is in the deflated position, also referred to herein as a collapsed position, physician 30 may insert catheter 22 into the body of patient 28 and may navigate distal-end assembly 40 to a target location, such as tissue 50 of heart 26.

In some embodiments, physician 30 may move shaft 23 in a direction opposite to a direction 90, so as to navigate distal-end assembly 40 to the target location. At the target location, physician 30 may inflate balloon 80 to the inflated position using a suitable fluid such as a saline solution, or using any other technique. After concluding the ablation procedure, physician 30 may move shaft 23 in direction 90 so as to retract distal-end assembly 40 out of the body of patient 28.

In some embodiments, when balloon 80 is in the inflated position, a distal end 60 of balloon 80 has a recessed shape shown as surfaces 100, which are substantially parallel to the target surface of tissue 50 as shown in Fig. 2A. Moreover, the surface of distal end 60 is perpendicular (on a planar side view) and specifically orthogonally to a longitudinal axis 66 of balloon 80, which is also the longitudinal axis of distal-end assembly 40.

In other embodiments, distal end 60 of balloon 80 may comprise a recessed shape at a nonzero angle relative to the surface of tissue 50. Additionally or alternatively, surfaces 100 of distal end 60 may not be perpendicular to longitudinal axis 66, but at any other suitable angle relative to axis 66.

In some embodiments, distal-end assembly 40 comprises one or more electrodes 88, in the present example six electrodes, disposed on an outer surface of balloon 80. In some embodiments, electrodes 88 are laid out along longitudinal axis 66 of distal-end assembly 40, but in other embodiments, electrodes 88 may be disposed on balloon 80 using any other suitable layout.

In some embodiments, due to the recessed shape of distal end 60 of balloon 80, respective regions (e.g., surfaces 100) of electrodes 88 become oriented perpendicularly to longitudinal axis 66 of balloon 80. In some embodiments, physician 30 may use distal-end assembly 40 for ablating tissue 50 using the region of electrodes 88 disposed on the recessed shape surface of distal end 60, e.g., surfaces 100 in the example of Fig. 2A. Note that in this configuration, distal-end 40 is configured to apply frontal ablation to the surface (also referred to herein as a wall) of tissue 50.

In some embodiments, shaft 23, which is coupled to an inner surface of balloon 80, comprises an inner lumen 70 that enables passage through the inner volume of balloon 80, along longitudinal axis 66. Before inserting catheter 22 into the patient body, an operator, such as physician 30, threads a guidewire 77 through inner lumen 70 of balloon 80. In the deflated position, balloon 80 is typically stored in a sheath (not shown) and is configured to move along guidewire 77, which is inserted through inner lumen 70.

During the ablation procedure, physician 30 navigates guidewire 77 to tissue 50, which is the target location in heart 26, using the position tracking system described in Fig. 1 above, or any other suitable navigation technique. Subsequently, physician 30 moves distal-end assembly 40, along guidewire 77, to tissue 50 or to any other target location.

In other embodiments, physician 30 may navigate distal-end assembly 40 to the target location without using a guidewire, for example, by having a three-dimensional (3D) position sensor coupled to distal-end assembly 40, as described in Fig. 1 above, and moving distal-end assembly 40 using shaft 23.

In some embodiments, power generator 48 is electrically coupled to each of electrodes 88 and is configured to supply respective amounts of power to electrodes 88. In some embodiments, the power generator distributes power among the electrode unevenly. For example, power generator 48 may supply a power level of 12Watts to one electrode 88 and a power level of 18Watts to another electrode 88 of distal-end assembly 40. In some embodiments, processor 34 is configured to produce an ablation plan and to determine, based on the ablation plan, the amount of power supplied to each electrode 88. In other words, based on the ablation plan, processor 34 sets the amount of ablation energy applied by the electrodes to respective areas of tissue 50, and the ablation start time and duration so as to produce the desired size and shape of a lesion within tissue 50.

In some embodiments, the ablation plan may specify, for each electrode 88 or for one or more selected groups of electrodes 88, suitable ablation parameters such as ablation time, contact force, ablation power, target temperature and/or any other suitable ablation parameters. In the context of the present invention, and in the claims, the terms "region" and "section" of electrode 88 are used interchangeably, and refer to different parts of electrode 88 located at different locations on balloon 80.

Note that the recessed shape of distal end 60, orients only surfaces 100, which are the respective regions of electrodes 88, perpendicularly to longitudinal axis 66 of balloon 80. In other words, for each electrode 88, only the section laid out within surfaces 100 of distal end 60, makes contact with the tissue and thus carries out the ablation. The sections of electrodes 88 are depicted in detail in Fig. 2B below.

In some embodiments, power generator 48 may apply to tissue 50, via all electrodes 88 of distal-end assembly 40, any suitable amount of total power. For example, a total power of 900Watts or any other suitable value between 20Watts and 900Watts can be used. As described above, processor 34 sets the power levels applied to tissue 50 by respective electrodes 88, which power levels sum up to the aforementioned total power determined by the parameters of the ablation plan.

### ABLATING HEART TISSUE USING DIFFERENT SECTIONS OF THE CATHETER ELECTRODES

Fig. 2B is a schematic, top view of distal-end assembly 40, in accordance with an embodiment of the present invention. The term "top view" refers to a view of distal-end assembly 40 from direction 90 shown in Fig. 2A above.

In some embodiments, each electrode 88 comprises at least two sections, referred to herein as sections 88A and 88B. Section 88A of each electrode 88 is disposed on balloon 80 within the recessed shape area of surfaces 100 of distal end 60, which are described in Fig. 2A above and shown as circles in Fig. 2B. Note that the ablation-related embodiments depicted in Fig. 2A above are all carried out by section 88A of electrodes 88. In some embodiments, in the aforementioned ablation plan, processor 34 holds information that is indicative of the shape and size of section 88A of each electrode 88, and the location of section 88A on the surface of balloon 80. The processor uses this information to calculate the amount of ablation power applied, via the respective section 88A, to tissue 50.

In some embodiments, the diameter of each surface 100 of distal end 60 may be about 2mm, or any other suitable size between 1mm and 10mm and the typical diameter of the entire area of distal end 60 may be about 6mm, or any other suitable size between 3mm and 30mmThe diameters of surfaces 100 and of distal end 60 are typically large relative to focal ablation catheters known in the art, which allows physician 30 to concurrently ablate large areas in tissue 50, or alternatively to ablate selected areas within this diameter by supplying power to a partial subset of electrodes 88. For example, processor 34 may send a control signal to power generator 48, to supply power only to two surfaces 100 from among all six surfaces 100 shown in Fig. 2B, thereby, only two respective areas of tissue 50 will be ablated, rather than six in case all electrodes 88 would have received the power.

Moreover, by ablating perpendicularly (e.g., orthogonally) to longitudinal axis 66, system 20 may accurately control the contact force between distal-end assembly 40 and tissue 50, in accordance with the ablation plan.

Section 88B of each electrode 88 is disposed on balloon 80, out of the recessed shape area of surfaces 100 of distal end 60. Therefore, in the ablation procedures carried out by distal end 60 and depicted in Fig. 2A above, only sections 88A are actually ablating tissue 50, whereas sections 88B of balloon 80 are not making physical contact with, and therefore are not ablating tissue 50.

In some embodiments, distal-end assembly 40 is further configured to carry out other types of ablation procedures, such as an oblique ablation. In the context of the present invention, and in the claims, the term "oblique ablation" refers to ablation of tissue, which is not perpendicular to the longitudinal axis of the ablating catheter.

For example, physician 30 may navigate distal-end assembly 40 to an ostium (not shown) of a pulmonary vein (PV) of heart 26. Subsequently, physician 30 may inflate balloon 80 so that sections 88B of electrodes 88 make physical contact with the ostium of the respective PV. Then, processor 34 applies a selected amount of power for each electrode 88 in accordance with a predetermined ablation plan, so as to carry out a PV isolation procedure in the selected PV.

In some embodiments, power generator 48 supplies the power independently to every electrode 88 or to a selected group of electrodes 88. Processor 34 determines the amount of power supplied by the power generator to each electrode 88 based on the calculated ablation plan. For example, processor 34 may determine a power level that is sufficiently high to form a continuous lesion along the entire ostium of the PV. Processor 34 typically limits the ablation duration and/or ablation power applied to the PV, and also limits the contact force applied to the ostium of the PV by section 88B of electrodes 88, so as to prevent anatomical damage to the PV, and/or to prevent formation of blood clots in the body of patient 28.

The particular configuration of distal-end assembly 40 is shown by way of example, in order to illustrate certain problems that are addressed by embodiments of the present invention and to demonstrate the application of these embodiments in enhancing the performance of such a cardiac ablation system. Embodiments of the present invention, however, are by no means limited to this specific sort of example distal-end assembly and/or system, and the principles described herein may similarly be applied to other sorts of catheters and ablation systems.

Fig. 3 is a schematic, side view of contours 40A and 40B of distal-end assembly 40, in accordance with embodiments of the present invention. Contours 40A and 40B are indicative of the shape of distal-end assembly 40 in the inflated and deflated positions, respectively.

As depicted in Figs. 2A and 2B above, when balloon 80 is in the inflated position, distal end 60 of assembly 40 has a recessed shape, shown as a flat section at the distal-end of contour 40A.

After concluding the ablation procedure, physician 30, or any other operator of system 20, deflates balloon 80 and pulls shaft 23 of catheter 22 in direction 90, so as to retract distal-end assembly 40 out of the body of patient 28. The deflation of balloon 80 may be carried out by allowing the balloon to evacuate saline into the blood pool, pumping the saline solution out of balloon 80, or using any other suitable deflating technique.

In some embodiments, in response to the deflating and pulling of balloon 80, distal-end assembly 40 is configured to fold to a deflated position by having the rounded edges at the side of shaft 23 (in the inflated position) folded along longitudinal axis 66 at the distal-end of balloon 80, as shown in contour 40B. The reduced cross-section of distal-end assembly 40, as shown by contour 40B, allows the insertion of distal-end assembly 40 into the sheath (not shown), so that physician 30 may retract catheter 22 out of the body of patient 28.

In some embodiments, a typical diameter of balloon 80 in the inflated position may be about 9mm, and in the deflated position, the diameter of balloon 80 is reduced to about 3mm.

In other embodiments, balloon 80 may have any other suitable diameter, for example, between 3mm and 35mm in the inflated position, and between 1mm and 5mm in the deflated position.

In other embodiments, distal-end assembly 40 may be designed to have any other suitable shape in the deflated position, so as to allow the insertion of distal-end assembly 40 into the sheath and to enable safe retraction of distal-end assembly 40 from the body of patient 28.

Although the embodiments described herein mainly address cardiac ablation, the methods and systems described herein can also be used in other applications, such as in otolaryngological or neurological ablation procedures.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

### ASPECTS OF THE INVENTION

1. A method, comprising:
   in a catheterization system that comprises an inflatable balloon for insertion into a patient organ, and one or more electrodes disposed on the balloon, wherein, when the balloon is in an inflated position, a distal end of the balloon has a recessed shape that orients respective regions of the electrodes perpendicularly to a longitudinal axis of the balloon, inserting the inflatable balloon into the patient organ;
   inflating the balloon to the inflated position for attaching the balloon to tissue of the patient organ; and
   ablating the tissue of the patient organ.
2. The method according to aspect 1, wherein the electrodes comprise at least a first electrode and a second electrode, and wherein ablating the tissue comprises applying a first power level to the first electrode and a second different power level to the second electrode.
3. The method according to aspect 1, wherein ablating the tissue comprises controlling power levels respectively supplied to the one or more electrodes.
4. The method according to aspect 1, wherein, in the inflated position, the electrodes have additional regions oriented not perpendicularly to the longitudinal axis, and wherein ablating the tissue comprises conducting an oblique ablation using the additional regions of the electrodes.
5. The method according to aspect 4, wherein the patient organ comprises a heart, and wherein conducting the oblique ablation comprises ablating an ostium of a pulmonary vein (PV) of the heart.
6. The method according to aspect 1, wherein the balloon comprises an inner lumen, and wherein inserting the inflatable balloon comprises moving the balloon along a guidewire inserted through the inner lumen.
7. The method according to aspect 1, and comprising folding the balloon along the longitudinal axis to a deflated position.

## Claims

1. A catheterization system, comprising:
an inflatable balloon for insertion into a patient organ; and
one or more electrodes disposed on the balloon, wherein, when the balloon is in an inflated position, a distal end of the balloon has a recessed shape that orients respective regions of the electrodes perpendicularly to a longitudinal axis of the balloon.

2. The catheterization system according to claim 1, and comprising a power generator, which is configured to supply electrical power to each of the one or more electrodes.

3. The catheterization system according to claim 2, wherein the electrodes comprise at least a first electrode and a second electrode, and wherein the power generator is configured to apply a first power level to the first electrode and a second different power level to the second electrode.

4. The catheterization system according to claim 2, and comprising a processor, which is configured to control power levels that the power generator respectively supplies to the one or more electrodes.

5. The catheterization system according to claim 1, wherein, when the distal end of the balloon makes physical contact with the organ, the respective regions of the electrodes, which are perpendicular to the longitudinal axis of the balloon, are configured to apply frontal ablation to a wall of the patient organ.

6. The catheterization system according to claim 1, wherein, in the inflated position, the electrodes have additional regions oriented not perpendicularly to the longitudinal axis, and wherein the additional regions of the electrodes are configured to conduct an oblique ablation to tissue of the patient organ.

7. The catheterization system according to claim 6, wherein the patient organ comprises a heart, and wherein the oblique ablation comprises ablation of an ostium of a pulmonary vein (PV) of the heart.

8. The catheterization system according to claim 1, wherein the balloon comprises an inner lumen, and wherein the balloon is configured to move along a guidewire inserted through the inner lumen.

9. The catheterization system according to claim 1, wherein the balloon is configured to fold along the longitudinal axis, to a deflated position.

10. A method for producing a catheterization system, the method comprising:
producing an inflatable balloon for insertion into a patient organ, wherein, when the balloon is in an inflated position, a distal end of the balloon has a recessed shape; and
disposing one or more electrodes on the balloon, such that the recessed shape orients respective regions of the electrodes perpendicularly to a longitudinal axis of the balloon.

11. The method according to claim 10, and comprising coupling to the one or more electrodes a power generator for supplying electrical power to the one or more electrodes.

12. The method according to claim 11, and comprising coupling to the power generator, a processor for controlling power levels that the power generator respectively supplies to the one or more electrodes.

13. The method according to claim 10, wherein, in the inflated position, the electrodes have additional regions oriented not perpendicularly to the longitudinal axis for conducting an oblique ablation to tissue of the patient organ.

14. The method according to claim 13, wherein the patient organ comprises a heart, and wherein the oblique ablation comprises ablation of an ostium of a pulmonary vein (PV) of the heart.

15. The method according to claim 10, wherein producing the balloon comprises producing, in the balloon, an inner lumen for moving the balloon along a guidewire inserted through the inner lumen.
